(12) **EUROPÄISCHE PATENTSCHRIFT**

| | |
|---|---|
| (45) Veröffentlichungstag und Bekanntmachung des Hinweises auf die Patenterteilung:<br>**19.05.2004   Patentblatt 2004/21** | (51) Int Cl.⁷: $A61B\ 17/70$ |
| (21) Anmeldenummer: **98943621.7** | (86) Internationale Anmeldenummer:<br>**PCT/CH1998/000415** |
| (22) Anmeldetag: **29.09.1998** | (87) Internationale Veröffentlichungsnummer:<br>**WO 2000/018310 (06.04.2000 Gazette 2000/14)** |

(54) **VORRICHTUNG ZUR VERBINDUNG EINES LÄNGSTRÄGERS MIT EINEM KNOCHENFIXATIONSMITTEL**

DEVICE FOR JOINING A LONGITUDINAL SUPPORT AND BONE FIXATION MEANS

DISPOSITIF POUR ASSEMBLER UN SUPPORT LONGITUDINAL ET UN MOYEN DE FIXATION D'OS

(84) Benannte Vertragsstaaten:
**AT BE CH DE DK ES FI FR GB GR IE IT LI NL PT SE**

(43) Veröffentlichungstag der Anmeldung:
**25.07.2001   Patentblatt 2001/30**

(73) Patentinhaber: **SYNTHES AG Chur**
**7002 Chur (CH)**

(72) Erfinder:
• **SCHLÄPFER, Fridolin**
**CH-8750 Glarus (CH)**

• **HESS, Martin**
**CH-4434 Hölstein (CH)**

(74) Vertreter: **Lusuardi, Werther Giovanni, Dr.**
**Dr. Lusuardi AG,**
**Kreuzbühlstrasse 8**
**8008 Zürich (CH)**

(56) Entgegenhaltungen:
**EP-A- 0 384 001      EP-A- 0 524 441**
**EP-A- 0 700 664      WO-A-88/01152**
**US-A- 5 451 225      US-A- 5 584 834**

**Beschreibung**

Die Erfindung bezieht sich auf eine Vorrichtung gemäss dem Oberbegriff des Patentanspruchs 1.

Aus dem Stand der Technik sind bereits einige Vorrichtungen zur Verbindung von Pedikelschrauben mit Längsträgern für die Wirbelsäulenfixation bekannt. Diese besitzen den Vorteil, dass die einzelnen Pedikelschrauben jederzeit am Längsträger befestigt oder wieder entfernt werden können, ohne dass das gesamte Fixationssystem demontiert werden muss. Auch kann beim Nachjustieren der Stellung der Verbindungsvorrichtung relativ zum Längsträger die Blockierung zwischen dem Kopf der Pedikelschraube und der Verbindungsvorrichtung erhalten werden.

Eine solche Verbindungsvorrichtung ist in der US 5,584,834 ERRICO offenbart. Diese bekannte Erfindung beinhaltet ebenfalls eine Vorrichtung, die es auf einfache Weise gestattet, eine Pedikelschraube oder allgemeiner ein Knochenverankerungselement mit einem Längsträger zu verbinden und die eine grosse Freiheit bezüglich des Winkels zwischen den beiden Elementen gestattet. Die Verbindungsvorrichtung besteht im wesentlichen aus einem zylinderförmigen Zentralkörper, welcher an seinem unteren Ende mit einer geschlitzten, aussen konischen Spannzange mit einer hohlkugelförmigen Kavität zur Aufnahme eines kugelförmigen Kopfes eines Knochenverankerungselementes versehen ist und an seinem oberen Ende ein Aussengewinde aufweist. In seinem Mittelteil ist dieser Zentralkörper mit einer quer zu seiner Zentralachse verlaufenden seitlich offenen Bohrung zur Aufnahme eines Längsträgers versehen. Über diesen Zentralkörper werden eine untere Hülse mit einem zum Konus des Zentralkörpers korrespondierenden Innenkonus und eine obere Hülse mit einer gegen die untere Hülse hin offenen Durchgangsöffnung geschoben. Beide Hülsen sind im montierten Zustand mittels einer Mutter, welche über das Aussengewinde am Zentralkörper schraubbar ist, gegen unten pressbar. Zwischen unterer und oberer Hülse wird der Längsträger eingeführt, welcher durch die Durchgangsöffnung in der oberen Hülse seitlich geführt wird. Beim Blockieren der Verbindungsvorrichtung wird beim Anziehen dieser Mutter die obere Hülse gegen unten gepresst, wodurch der in der Durchgangsöffnung eingeführte Längsträger auf die untere Hülse drückt, deren Innenkonus in der Folge über den Aussenkonus der Spannzange geschoben wird. Das Übereinanderschieben der Konusse bewirkt ein Komprimieren der Spannzange und somit eine Blockierung des darin eingespannten Kugelkopfes.

Nachteilig bei dieser in der US 5,584,834 offenbarten Verbindungsvorrichtung ist, dass beim Anziehen der Mutter der wegen der seitlich offenen Durchgangsöffnung exzentrisch die Zugkraft aufnehmende Zentralkörper aufgebogen wird und dadurch die Spannkraft auf den Längsträger zu gering ist, unter Belastung ein Federn des Zentralkörpers auftritt oder auf Grund des exzentrischen Druckes auf die untere Hülse eine Verkanten der Hülse eintreten kann. Durch das Aufbiegen des Zentralkörpers hat der Längsträger die Tendenz aus der seitlichen Durchgangsöffnung im Zentralkörper zu rutschen mit der Folge, dass die obere Hülse sich aufbiegt. Dieses Problem konnte bei besagter Konstruktion nur behoben werden, indem in der unteren Hülse eine Führungsnut für den Längsträger angebracht wurde. Diese Nut bedingt aber, dass bei der Montage die untere Hülse ausgerichtet werden muss, was leicht zu Montagefehlern führt. Ferner besteht bei einer Spannzange, welche durch das Überschieben eines Innenkonusses über einen Aussenkonus komprimiert wird und dadurch den in der Spannzange eingeführten kugelförmigen Kopf des Knochenverankerungselementes blockiert, die Gefahr eines undefinierten verkanteten Verklemmens der Spannzange, welches durch nicht ganz genau übereinstimmende Konuswinkel verursacht wird. Zudem verändert sich bei einseitig befestigten Zungen der Spannzange der Konuswinkel der Spannzange während der Kompression, so dass die Schliesskraft der Konusverbindung im hinteren, bei der Befestigung der Zungen gelegenen Bereich der Konusverbindung übertragen wird. Diese ungünstige Kraftübertragung auf die Zungen, welche im innerhalb der Zungen liegenden Hohlraum den kugelförmigen Kopf des Knochenfixationsmittels umfassen, bewirkt ein Festklammern dieses kugelförmigen Kopfes derart, dass der kugelförmige Kopf durch die axiale Komponente der Schliesskraft eine Kraft erfährt, welche den kugelförmigen Kopf gegen die nicht mehr im Konus geführten Spitzen der Zungen presst.

Hier will die Erfindung Abhilfe schaffen. Das Ziel der Erfindung liegt darin, eine Vorrichtung zur Verbindung eines Längsträgers mit einem Knochenverankerungsmittel mit folgenden Eigenschaften zu schaffen:

- Aufbringen der Druckkraft auf die die Spannzange komprimierende, untere Hülse in einer senkrecht zur Zentralachse stehenden Ebene:

  . Verhinderung des Verkantens der Hülse;
  . Verhinderung des Aufbiegens des Zentralkörpers und damit Verhinderung, dass der Längsträger aus der seitlich offenen Durchgangsöffnung rutscht.

Die Erfindung löst die gestellte Aufgabe mit einer Vorrichtung, welche die Merkmale des Anspruchs 1 aufweist.

In einer bevorzugten Ausführungsform umfasst die erfindungsgemässe Vorrichtung einen zylinderförmigen Körper mit einer nach unten offenen hohlkugelförmigen und komprimierbaren Kammer für die Aufnahme eines kugelförmigen Kopfes eines Knochenfixationsmittels, einen konzentrisch zum Körper angeordneten unteren Hohlzylinder, einen ebenfalls konzentrisch zum Körper angeordneten oberen Hohlzylinder und eine Spannmutter, welche über ein Aussengewinde am oberen Ende des Körpers schraubbar ist. Im Bereich der Kammer ist der Körper aussen kugelförmig gestaltet und die Aussenwand der Kammer mit gegen das untere Ende des Körpers hin offenen Schlitzen versehen, wodurch die Komprimierbarkeit der Kammer hergestellt wird. Die Kammer weist einen Durchmesser d auf und ist als Kugelschicht mit einer Höhe x < d ausgebildet. Die senkrecht zur Zentralachse stehende Grosskreisebene der Kugelschicht liegt innerhalb der Kammer in einem Abstand y < x vom oberen Ende der Kammer, wodurch gewährleistet ist, dass der kugelförmige Kopf des Knochenfixationsmittels in die Kammer einschnappbar ist und nach dem Einschnappen nur durch Aufbringen einer Zugkraft wieder aus der Kammer entfernt werden kann. Die im Bereich der Kammer liegende kugelförmige Aussengestalt des Körpers ist ebenfalls kugelschichtartig. Jedoch liegt das Zentrum dieser kugelschichtförmigen Aussenfläche der Körpers unterhalb des Zentrums der Kugelschicht der Kammer.

Der als Klemmring für die Kammer wirkende untere Hohlzylinder wird nachh unten weiter und wird beim Komprimieren der Kammer über die kugelschichtförmige Aussenfläche des Körpers geschoben. Beim konzentrischen Überschieben dieses unteren Hohlzylinders entsteht daher ein nur kreislinienförmiger Kontakt zur kugelförmigen Aussenwand des Körpers. Dadurch wird eine genau definierte Position dieser Kontaktzone und somit ein gleichmässiges Komprimieren der Kammer erreicht.

In seinem zwischen der unten liegenden Kammer und dem oben angebrachten Aussengewinde liegenden zylindrischen Mittelteil weist der Körper eine quer und exzentrisch zur Zentralachse verlaufende, seitlich offene Bohrung zur Aufnahme des Längsträgers auf. Der obere Hohlzylinder ist mit einer zur Bohrung im Körper korrespondierenden, nach unten offenen ebenfalls quer zur Zentralachse verlaufenden Durchgangsöffnung zur Aufnahme des Längsträgers versehen. Diese Durchgangsöffnung umfasst den Längsträger nur partiell von oben her. Beim Anziehen der Spannmutter wird der obere Hohlzylinder nach unten gepresst und drückt auf den in der Bohrung und der Durchgangsöffnung eingelegten Längsträger, welcher wiederum auf den unteren Hohlzylinder drückt und diesen nach unten schiebt, wodurch einerseits der Längsträger zwischen dem oberen und dem unteren Hohlzylinder festgeklemmt und andererseits die Kammer mit dem darin gelagerten kugelförmigen Kopf des Knochenfixationsmittles komprimiert wird.

Durch die Durchgangsöffnung wird der obere Hohlzylinder in zwei Mantelsektoren aufgeteilt, wobei der obere Hohlzylinder so gestaltet ist, dass diese beiden Mantelsektoren eine unterschiedliche Höhe aufweisen. Dabei weist derjenige Sektor, welcher zum Verschluss der seitlich offenen Bohrung des Körpers bestimmt ist, eine geringere Höhe als der diametral gegenüberliegende Mantelsektor auf. Die Höhe H des höheren Mantelsektors entspricht der Summe H = D + A, wobei D dem Durchmesser des Längsträgers und A dem Abstand zwischen dem Dach der Durchgangsöffnung und der oberen Grundfläche des oberen Hohlzylinders entspricht. Zudem ist die Durchgangsöffnung exzentrisch zur Zentralachse so angeordnet, dass derjenige Mantelsektor, welcher zum Verschluss der seitlich offenen Bohrung des Körpers bestimmt ist, sektoriell kleiner ist als der diametral gegenüberliegende Mantelsektor. Durch diese Anordnung von verschieden hohen Mantelsektoren wird erreicht, dass neben dem Längsträger auch der Höhere der Mantelsektoren beim Anziehen der Spannmutter auf den unteren Hohlzylinder drückt. Da die Höhe H der Summe H = D + A entspricht, drücken der höhere Mantelsektor und der Längsträger in einer senkrecht zur Zentralachse stehenden Ebene gleichmässig auf den unteren Hohlzylinder, wodurch dieser koaxial über die kugelförmige Aussenfläche des Körpers geschoben wird und somit die Kammer exakt radial komprimiert wird.

Durch die Ausgestaltung der bezüglich der Zentralachse diametral zum Längsträger liegenden, sektoriell grösseren Mantelfläche in der Art, dass die Höhe H nur in einem symmetrisch zur Durchgangsöffnung angeordneten Sektorbereich $\beta$ von 10° bis 20° der Summe H = D + A entspricht, ist die Aufteilung des Kraftflusses über den Längsträger und die sektoriell grössere Mantelfläche steuerbar. Das Verhältnis der Aufteilung des Kraftflusses ist vorzugsweise 1:3, so dass 75% der durch das Spannmittel erzeugten Kompressionskraft über den Längsträger und 25% über den Sektorbereich mit der höheren Mantelfläche H = D + A fliesst. Die über den Längsträger fliessende Kraft bewirkt dessen Klemmung. Zudem ist die Höhe des unteren Hohlzylinders derart bemessen, dass der in der Bohrung und in der Durchgangsöffnung eingelegte Längsträger den Boden der Bohrung beim Anziehen der Spannmutter nicht erreichen kann. Die unterschiedliche Höhe der beiden Mantelsektoren liegt dabei vorzugsweise im Bereich von 0,3 - 1,0 mm.

Die Kammer ist in ihrem Inneren so ausgestaltet, dass der am kugelförmigen Kopf anschliessende Schaft einer Pedikelschraube innerhalb eines Winkels von $\alpha$ von - 25° bis + 25° gegenüber der Zentralachse blockierbar ist. Dies führt zu einer erheblichen Erweiterung des Anwendungsbereichs der erfindungsgemässen Vorrichtung.

Ferner ist das die Kammer enthaltende Unterteil des Körpers von dessen zylinderförmigen Mittelteil durch einen ringförmigen Ansatz getrennt und der untere Hohlzylinder am inneren Umfang zum Ansatz korrespondierend mit Lamellen versehen, deren Innendurchmesser kleiner als der Durchmesser das ringförmigen Ansatzes ist, so dass der untere Hohlzylinder von oben über den Körper einschiebbar ist und die Lamellen über den ringförmigen Ansatz einschnappbar sind. Dadurch wird das Aufschieben der als Klemmring dienenden Hülse in situ mit einschnappbar wirkender Sicherung gegen nachträgliches unerwünschtes Herausrutschen erreicht. Damit wird ein Abklicken des Zentralkörpers von dem kugelförmigen Kopf des Knochenfixationsmittel auch unter Krafteinwirkung verhindert.

Der Kopf eines zur Vorrichtung gehörenden Knochenfixationsmittels sowie die kugelförmige Kammer sind mit Vorteil mit einer Strukturierung versehen. Zusätzlich kann der Kopf des Knochenfixationsmittels im Bereich der Strukturierung der Kammer eine Zone aufweisen, welche weicher als die strukturierte Oberfläche der Kammer ist. Beide dieser beschriebenen Massnahmen bewirkten eine Erhöhung der rotativen Blockierung des Kopfes in der Kammer.

Die Strukturierung am kugelförmigen Kopf des Knochenfixationsmittels und/oder in der Kammer kann beispielsweise mittels einer Aufrauhung in Form von Rillen erfolgen. Der beschriebene Härteunterschied kann durch unterschiedliche Kaltverformung, durch unterschiedliche Auskristallisation des gleichen Materials oder durch die Wahl von unterschiedlichen Materialien erreicht werden, wobei das harte Material vorzugsweise hochtrainierter 1.4441 Stahl beziehungsweise eine harte Titanlegierung (TAN, TAV) ist und das weiche Material warmbehandelter 1.4441 Stahl beziehungsweise weicher Reintitan ist. Daneben kann der Härteunterschied durch Oberflächenbehandlung wie Beschichtung oder Ionenimplantation erreicht werden. Möglich ist auch eine Bestrahlung eines oder beider der in Kontakt stehenden Teile mit Korund.

Um eine Pedikelschraube in den Knochen eindrehen zu können, ist diese im Kugelkopf vorzugsweise mit einem Innensechskant versehen. Wenn der aufklickbare Körper der Verbindungsvorrichtung noch durchbohrt ist, kann wahlweise nur die Pedikelschraube oder gleich die gesamte Vorrichtung eingedreht werden. Letzteres hat vor allem den Vorteil, dass jederzeit die Vorrichtung weiter eingedreht oder zurückgedreht werden kann, um einen Höhenausgleich zu erreichen.

In einer weiteren bevorzugten Ausführungsform der erfindungsgemässen Vorrichtung weist die als Klemmring dienende untere Hülse innen einen nach unten weiter werdenden Konus auf, welcher beim Komprimieren der Kammer über die kugelschichtförmige Aussenfläche des Körpers geschoben wird. Damit ergibt sich eine nahezu kreislinienförmige Auflage des Innenkonusses in der unteren Hülse auf der Aussenfläche der Spannzange, womit eine bevorzugte definierte Kontaktzone beim Blockieren des kugelförmigen Kopfes des Kochenverankerungsmittels in der Spannzange hergestellt wird.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der linienförmigen Auflage zwischen dem Innenkonus des als Klemmring dienenden unteren Hohlzylinders und der kugelförmigen Aussenfläche des Körpers im Bereich der Kammer eine genau definierte Kontaktzone entsteht und damit eine gleichmässige Klemmung des Kugelkopfes erreicht wird. Ferner wird durch die Ausgestaltung der unterschiedlich hohen Mantelflächensektoren des oberen Hohlzylinders erreicht, dass die beim Anziehen der Spannmutter auf den oberen Hohlzylinder ausgeübte Druckkraft durch den Längsträger einerseits und den höheren Mantelflächensektor andererseits auf drei über die Stirnfläche des unteren Hohlzylinders verteilten Auflageflächen auf den unteren Hohlzylinder übertragen wird. Dadurch ist ein exakt koaxiales Schieben des unteren Hohlzylinders nach unten gewährleistet. Ferner wird durch die Lamellen am inneren Umfang des unteren Hohlzylinders und dem dazu korrespondierenden ringförmigen Ansatz am Körper die Möglichkeit des Aufschiebens des unteren Hohlzylinders in situ mit einschnappbar wirkender Sicherung gegen nachträgliches unerwünschtes Zurückrutschen des unteren Hohlzylinders erreicht. Die Sicherung des unteren Hohlzylinders ermöglicht die Übertragung grosser Kräfte über das Knochenfixationsmittel auf die Wirbelsäule, ohne dass ein Abklicken des Zentralkörpers von dem in der Kammer positionierten kugelförmigen Kopf des Knochenfixationsmittels auftritt.

Die bevorzugte Ausführungsform der Erfindung wird im folgenden anhand der teilweise schematischen Darstellungen noch näher erläutert.

Es zeigen:

Fig. 1 einen Längsschnitt durch die bevorzugte Ausführungsform der erfindungsgemässen Vorrichtung zusammen mit einem Längsträger und einer Knochenschraube als Knochenfixationsmittel mit Kugelkopf;

Fig. 2 einen Längsschnitt durch den mit der Kammer versehenen Körper und den unteren Hohlzylinder der in Fig. 1 dargestellten Ausführungsform der erfindungsgemässen Vorrichtung;

Fig. 3 eine Detailansicht der Kontaktzone zwischen dem im unteren Hohlzylinder angebrachten Innenkonus und der kugelförmigen Aussenwand des Körpers;

Fig. 4 eine Ansicht des oberen Hohlzylinders von unten;

Fig. 5 einen Aufriss des oberen Hohlzylinders; und

Fig. 6 die Aufteilung des Kraftflusses auf den Längsträger und den oberen Hohlzylinder.

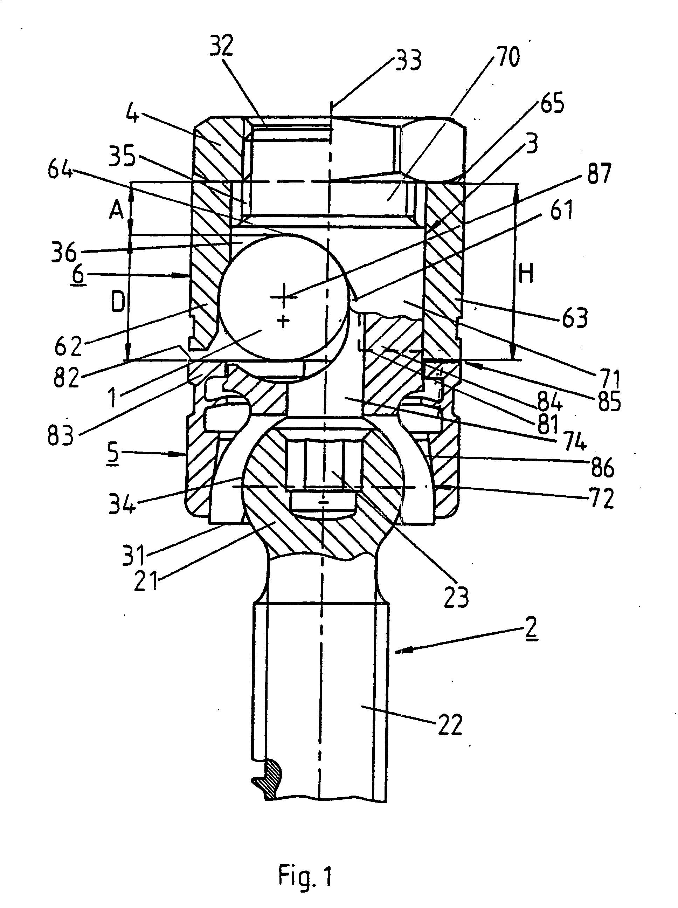

In der in Fig. 1 gezeigten bevorzugten Ausführungsform umfasst die erfindungsgemässe Vorrichtung einen Körper (3) mit einer nach unten offenen, hohlkugelförmigen und komprimierbaren Kammer (34) für die Aufnahme des kugelförmigen Kopfes (21) eines Knochenfixationsmittels (2), einen konzentrisch zum Körper (3) angeordneten unteren Hohlzylinder (5), einen ebenfalls konzentrisch zum Körper (3) angeordneten oberen Hohlzylinder (6) und eine Spannmutter (4). Zum Blockieren von Längsträger (1) und kugelförmigen Kopf (21) wird diese Spannmutter (4) über das Aussengewinde (35) am Oberteil (70) des Körpers (3) geschraubt und angezogen. Im Unterteil (72) des Körpers (3), welcher im Bereich der Kammer (34) liegt, ist der Körper (3) aussen kugelförmig gestaltet. In seinem zwischen der unten liegenden Kammer (34) und dem Oberteil (70) liegenden zylindrischen Mittelteil (71) weist der Körper (3) eine quer und exzentrisch zur Zentralachse (33) verlaufende, seitlich offene Bohrung (36) zur Aufnahme des Längsträgers (1) auf. Der obere Hohlzylinder (6) ist mit einer zur Bohrung (36) im Körper (3) korrespondierenden, nach unten offenen ebenfalls quer zur Zentralachse (33) verlaufenden Durchgangsöffnung (61) zur Aufnahme des Längsträgers (1) versehen. Diese Durchgangsöffnung (61) umfasst den Längsträger nur partiell von oben her. Beim Anziehen der Spannmutter (4) wird der obere Hohlzylinder (6) nach unten gepresst und drückt auf den in der Bohrung (36) und der Durchgangsöffnung (61) eingelegten Längsträger (1), welcher wiederum auf den unteren Hohlzylinder (5) drückt und diesen nach unten schiebt. Durch die Durchgangsöffnung (61) wird der obere Hohlzylinder (6) in zwei Mantelsektoren (62;63) aufgeteilt, wobei der obere Hohlzylinder (6) so gestaltet ist, dass diese beiden Mantelsektoren (62;63) eine unterschiedliche Höhe aufweisen. Dabei weist derjenige Sektor (62), welcher zum Verschluss der seitlich offenen Bohrung (36) des Körpers (3) bestimmt ist, eine geringere Höhe als der diametral gegenüberliegende Mantelsektor (63) auf. Die grössere Höhe des Mantelsektors (63) wird durch Anbringen einer sektoriellen Erhebung (80) mit einem Winkel von 20° an der unteren Stirnfläche (81) des oberen Hohlzylinders (6) hergestellt. Die somit erreichte Höhe H des höheren Mantelsektors (63) entspricht der Summe H = D + A, wobei D dem Durchmesser des Längsträgers (1) und A dem Abstand zwischen dem Dach (64) der Durchgangsöffnung (61) und der oberen Grundfläche (65) des oberen Hohlzylinders (6) entspricht. Zudem ist die Durchgangsöffnung (61) exzentrisch zur Zentralachse (33) so angeordnet, dass derjenige Mantelsektor (62), welcher zum Verschluss der seitlich offenen Bohrung (36) des Körpers (3) bestimmt ist, sektoriell kleiner ist als der diametral gegenüberliegende Mantelsektor (63). Durch diese Anordnung von verschieden hohen Mantelsektoren (62;63) wird erreicht, dass neben dem Längsträger (1) auch der Höhere der Mantelsektoren (63) beim Anziehen der Spannmutter (4) auf den unteren Hohlzylinder (5) drückt.

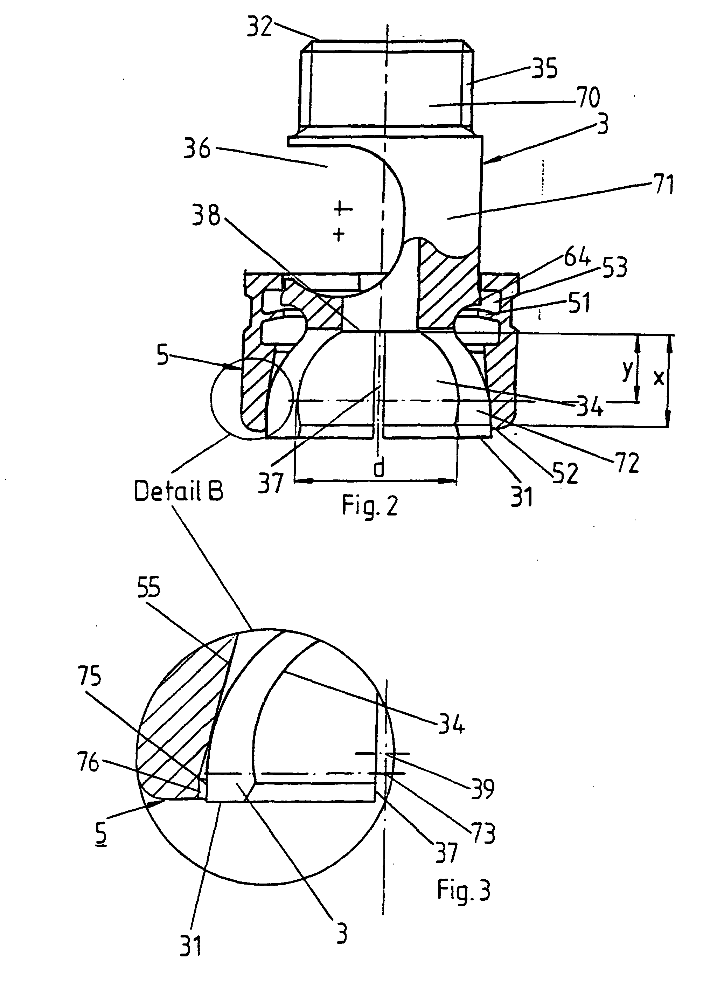

Wie in Fig. 2 gezeigt weist die Kammer (34) einen Durchmesser d auf und ist als Kugelschicht mit einer Höhe x < d ausgebildet. Die senkrecht zur Zentralachse (33) stehende Grosskreisebene der Kugelschicht liegt innerhalb der Kammer (34) in einem Abstand y < x vom oberen Ende (38) der Kammer (34). Der im Bereich der Kammer (34) liegende Unterteil (72) des Körpers (3) ist ebenfalls kugelschichtartig. Jedoch liegt das Zentrum (73) dieses kugelschichtförmigen Unterteils (72) unterhalb des Zentrums (39) der Kugelschicht der Kammer (34). Das die Kammer (34) enthaltende Unterteil (72) des Körpers (3) ist von dessen zylinderförmigen Mittelteil (71) durch einen ringförmigen Ansatz (64) getrennt und der untere Hohlzylinder (5) am inneren Umfang zum Ansatz (64) korrespondierend mit Lamellen (51) versehen, deren Innendurchmesser kleiner als der Durchmesser das ringförmigen Ansatzes (64) ist, so dass der untere Hohlzylinder (5) vom oberen Ende (32) über den Körper (3) schiebbar ist und die Lamellen (51) über den ringförmigen Ansatz (64) einschnappbar sind.

Fig. 3 zeigt einen Ausschnitt aus dem Unterteil (72) des Körpers (3), wobei die Aussenwand der Kammer (34) mit gegen das untere Ende (31) des Körpers (3) hin offenen Schlitzen (37) versehen ist. Damit wird eine Komprimierbarkeit der Kammer (34) erreicht. Der als Klemmring für die Kammer (34) wirkende untere Hohlzylinder (5) weist innen einen nach unten weiter werdenden Konus (55) auf, welcher über die kugelschichtförmige Aussenfläche des Körpers (3) geschoben wird und die Kammer (34) komprimiert. Beim konzentrischen Überschieben dieses unteren Hohlzylinders (5) entsteht daher ein nur kreislinienförmiger Kontakt zur kugelförmigen Aussenwand des Körpers (3). Der untere Hohlzylinder (5) ist unten innen und der Unterteil (72) aussen am unteren Ende (31) zylindrisch ausgebildet, wobei die Durchmesser, die Höhe und die Position dieser zylindrischen Teile (75;76) derart aufeinander abgestimmt sind, dass bei nicht angezogenem Spannmittel (4) ein Aufweiten des Unterteils (72) durch den zylindrischen Teil (76) des unteren Hohlzylinders (5) verhindert wird. Beim Manipulieren des Implantates ohne eingeschobenen Längsträger (1) (z.B. Hochheben des Implantates zum Längsträger (1)) kann auf Grund des Längsspiels zwischen den Lamellen (51) und dem Absatz (64) der untere Hohlzylinder (5) so weit zurückrutschen oder zurückgedrückt werden, dass der Konus (55) nicht mehr auf der kugelförmigen Aussenfläche der Unterteils (72) aufliegt und dementsprechend ein Aufweiten des Unterteils (72) nicht mehr verhindert. Damit besteht ohne die zylindrischen Teile (75;76) bei Zug auf die Knochenschraube (2) die Gefahr des Ausklickens des Kugelkopfes (21).

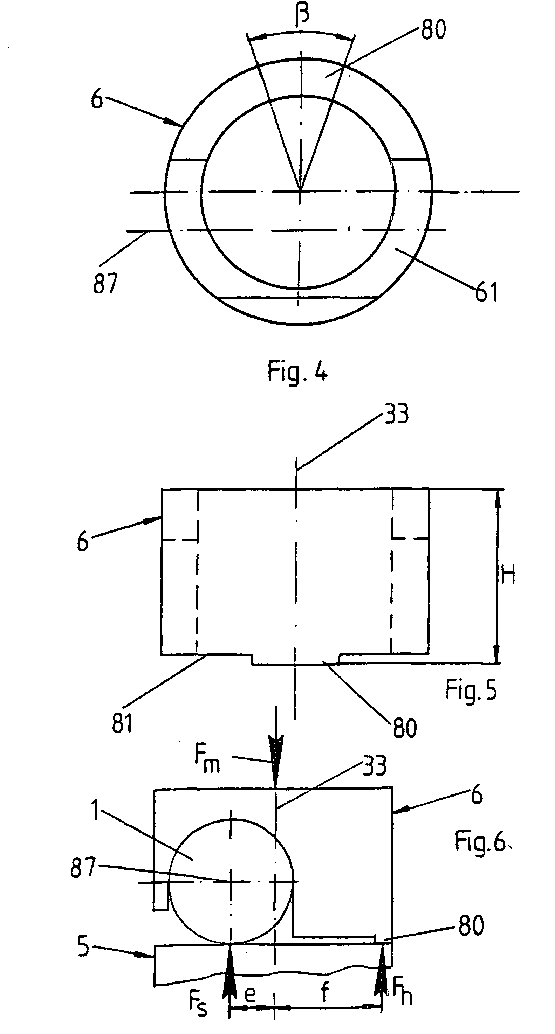

Fig. 4 und 5 zeigen den oberen Hohlzylinder (6) mit der exzentrisch zur Zentralachse (33) liegenden Durchgangsöffnung (61) und der auf der unteren Stirnfläche (81) im Sektorbereich β angebrachten Erhebung (80), so dass nur im Sektorbereich β die grössere Höhe H des Mantelsektors (63) vorherrscht.

Fig. 6 zeigt die auf den Längsträger (1) und auf den oberen Hohlzylinder (6) wirkenden resultierenden Kräfte beim Anziehen des Spannmittels (4) (nicht gezeichnet). Dabei ist $F_m$ die resultierende Kraft des Spannmittels (4), $F_s$ die resultierende Kraft des unteren Hohlzylinders (5) auf den Längsträger (1) und $F_h$ die resultierende Kraft des unteren Hohlzylinders (5) auf die im Sektorbereich β liegende Erhebung (80). Auf dem senkrecht zur Längsachse (87) der Durchgangsöffnung (61) stehenden Durchmesser des oberen Hohlzylinders (6) ergibt sich zwischen dem Angriffspunkt der resultierenden Kraft $F_s$ und der Zentralachse (33) ein Abstand (e), während ebenfalls auf dem senkrecht zur Längsachse (87) der Durchgangsöffnung (61) stehenden Durchmesser zwischen dem Angriffspunkt der resultierenden Kraft $F_h$ und der Zentralachse (33) ein Abstand (f) vorliegt. Bei kreiszylindrischen Längsträgern (1) entspricht der Abstand (e) auch dem Abstand zwischen der Längsachse (87) der Durchgangsöffnung (61) und der Zentralachse (33). Eine Betrachtung des Kräfte- und Momentengleichgewichts ergibt für $F_h$ bezüglich $F_m$ den folgenden Zusammenhang:

$$F_s = \frac{F_m}{1 + e/f}$$

Damit ergibt sich für ein Verhältnis von e/f von 1 : 3, welches beispielsweise durch die Ausführung einer oberen Hülse (6) mit den Abmessungen e = 2 mm und f = 6 mm gegeben ist, ein Verhältnis für $F_s / F_m$ von 0,75.

## Patentansprüche

1. Vorrichtung zur Verbindung eines Längsträger (1) mit einem einen kugelförmigen Kopf (21) aufweisenden Knochenfixationsmittel (2) innerhalb einer Wirbelsäulenfixationsvorrichtung, mit

   A) einem zylinderförmigen Körper (3) mit einer Zentralachse (33), welcher ein Oberteil (70) mit einem oberen Ende (32), ein Mittelteil (71) und ein Unterteil (72) mit einem unteren Ende(31) umfasst, wobei

   a) das Unterteil (72) mit einer nach unten offenen, kugelförmigen, komprimierbaren Kammer (34) für die Aufnahme des kugelförmigen Kopfes (21) versehen ist;
   b) das Oberteil (70) Mittel (35) zur Aufnahme eines Spannmittels (4) für den Längsträger (1) aufweist; und
   c) das Mittelteil (71) eine quer zur Zentralachse (33) verlaufende Bohrung (36) zur Aufnahme des Längsträgers (1) aufweist;

   B) einem konzentrisch zum Körper (3) angeordneten unteren Hohlzylinder (5), welcher vom oberen Ende (32) her über das Unterteil (72) schiebbar ist, wobei der minimale Innendurchmesser des unteren Hohlzylinders (5) kleiner als der maximale Aussendurchmesser des Unterteils (72) ist, so dass das Unterteil (72) mit der Kammer (34) durch den nach unten schiebbaren unteren Hohlzylinder (5) komprimierbar und ein sich in der Kammer (34) befindender kugelförmiger Kopf (21) rotativ festklemmbar ist;
   C) einem konzentrisch zum Körper (3) angeordneten oberen Hohlzylinder (6) mit einer zur Bohrung (36) korrespondierenden, nach unten offenen, quer zur Zentralachse (33) verlaufenden, zur partiellen Umfassung des Längsträgers(1) bestimmten Durchgangsöffnung (61); wobei
   D) der untere Hohlzylinder (5) mittels eines in der Bohrung (36) und Durchgangsöffnung (61) eingelegten Längsträgers (1) durch die Wirkung des Spannmittels (4) auf den oberen Hohlzylinder (6) nach unten schiebbar ist, **dadurch gekennzeichnet, dass**
   E) am unteren Ende (84) des oberen Hohlzylinders (6) und/oder am oberen Ende (83) des unteren Hohlzylinders (5) Mittel (85) vorgesehen sind, welche zwischen den benachbarten Stirnflächen (81;82) des oberen (6) und des unteren Hohlzylinders (5) neben zwei Berührungsstellen eines in der Bohrung (36) und Durchgangsöffnung (61) eingelegten Längsträgers (1) mindestens eine dritte Berührungsstelle bilden, so dass durch die Wirkung des Spannmittels (4) auf den oberen Hohlzylinder (6) der untere Hohlzylinder (5) zur Zentralachse (33) koaxial nach unten schiebbar ist; und
   F) die Aussenwand (86) des Unterteils (72) kugelzonenförmig ausgebildet ist.

2. Vorrichtung nach Anspruch 1, **dadurch gekennzeichnet, dass** die Aussenwand (86) des Unterteils (72) konvex ausgebildet ist.

3. Vorrichtung nach Anspruch 1 oder 2, **dadurch gekennzeichnet, dass** die Mittel (85) so angeordnet sind, dass durch die zwei Berührungsstellen eines in der Bohrung (36) und Durchgangsöffnung (61) eingelegten Längsträgers (1) und die mindestens eine dritte Berührungsstelle der Mittel (85) ein in einem minimalen Abstand zur Zentralachse (33) liegender resultierender Kraftangriffspunkt entsteht.

4. Vorrichtung nach einem der Ansprüche 1 bis 3, **dadurch gekennzeichnet, dass** die Bohrung (36) zur Aufnahme des Längsträgers (1) exzentrisch zur Zentralachse (33) im Körper (3) angebracht ist.

5. Vorrichtung nach einem der Ansprüche 1 bis 4, **dadurch gekennzeichnet, dass** die Bohrung (36) am Körper (3) seitlich offen ist.

**6.** Vorrichtung nach einem der Ansprüche 1 bis 5, **dadurch gekennzeichnet, dass** die Mittel (85) darin bestehen, dass die Durchgangsöffnung (61) den oberen Hohlzylinder (6) in zwei Mantelsektoren (62,63) mit unterschiedlicher Höhe aufteilt, wobei derjenige Mantelsektor (62), welcher zum Verschluss der seitlich offenen Bohrung (36) des Körpers (3) bestimmt ist, eine geringere Höhe als der diametral gegenüberliegende Mantelsektor (63) aufweist.

**7.** Vorrichtung nach Anspruch 6, **dadurch gekennzeichnet, dass** der Mantelsektor (63) des oberen Hohlzylinders (6) mindestens in einem symmetrisch zur Durchgangsöffnung (61) angeordneten Sektorbereich β von 10° - 20° eine grössere Höhe als derjenige Mantelsektor (62) aufweist, welcher zum Verschluss der seitlich offene Bohrung (36) des Körpers (3) bestimmt ist.

**8.** Vorrichtung nach Anspruch 6 oder 7, **dadurch gekennzeichnet, dass** die unterschiedliche Höhe der beiden Mantelsektoren (62,63) im Bereich von 0,3 - 1,0 mm liegt.

**9.** Vorrichtung nach einem der Ansprüche 4 bis 8, **dadurch gekennzeichnet, dass** die Durchgangsöffnung (61) exzentrisch zur Zentralachse (33) angeordnet ist, so dass derjenige Mantelsektor (62), welcher zum Verschluss der seitlich offenen Bohrung (36) des Körpers (3) bestimmt ist, sektoriell kleiner ist als der diametral gegenüberliegende Mantelsektor (63).

**10.** Vorrichtung nach einem der Ansprüche 1 bis 9, **dadurch gekennzeichnet, dass** die innere Mantelfläche des unteren Hohlzylinders (5) eine von der Aussenwand (86) des Unterteils (72) abweichende Form aufweist, so dass beim konzentrischen Überschieben des Hohlzylinders (5) nur ein kreislinienförmiger Kontakt zur Aussenwand (86) des Unterteils (72) erfolgt.

**11.** Vorrichtung nach einem der Ansprüche 1 bis 9, **dadurch gekennzeichnet, dass** die innere Mantelfläche des unteren Hohlzylinders (5) eine von der Aussenwand (86) des Unterteils (72) abweichende Form aufweist, so dass beim konzentrischen Überschieben des Hohlzylinders (5) nur ein kreiszonenförmiger Kontakt zur Aussenwand (86) des Unterteils (72) erfolgt.

**12.** Vorrichtung nach Anspruch 11, **dadurch gekennzeichnet, dass** die Zonenhöhe der kreiszonenförmigen Kontaktfläche zwischen der inneren Mantelfläche des unteren Hohlzylinders (5) und der Aussenwand (86) des Unterteils (72) maximal 2 mm beträgt.

**13.** Vorrichtung nach einem der Ansprüche 1 bis 12, **dadurch gekennzeichnet, dass** die Höhe des unteren Hohlzylinders (5) derart bemessen ist, dass der in der Bohrung (36) und Durchgangsöffnung (61) eingelegte Längsträger (1) den Boden der Bohrung (36) bei Betätigung des Spannmittels (4) nicht erreichen kann.

**14.** Vorrichtung nach einem der Ansprüche 1 bis 13, **dadurch gekennzeichnet, dass** das Zentrum (73) der kugelzonenförmigen Aussenwand des Unterteils (72) unterhalb des Zentrums (39) der kugelförmigen Kammer (34) liegt, wobei unterhalb näher gegen das untere Ende (31) des Körpers (3) bedeutet.

**15.** Vorrichtung nach einem der Ansprüche 1 bis 14, **dadurch gekennzeichnet, dass** das Mittel (35) ein Aussengewinde ist, welches eine Mutter als Spannmittel (4) aufnehmen kann.

**16.** Vorrichtung nach einem der Ansprüche 1 bis 14, **dadurch gekennzeichnet, dass** das Mittel (35) ein Innengewinde ist, welches eine Schraube als Spannmittel (4) aufnehmen kann.

**17.** Vorrichtung nach einem der Ansprüche 1 bis 16, **dadurch gekennzeichnet, dass** das Knochenfixationsmittel (2) eine Knochenschraube oder ein Haken ist.

**18.** Vorrichtung nach einem der Ansprüche 1 bis 17, **dadurch gekennzeichnet, dass** die Kammer (34) derart ausgebildet ist, dass der am kugelförmigen Kopf (21) anschliessende Schraubenschaft (22) der Knochenschraube (2) in einem Winkel α von -25° bis + 25° gegenüber der Zentralachse (33) blokkierbar ist.

**19.** Vorrichtung nach einem der Ansprüche 1 bis 18, **dadurch gekennzeichnet, dass** der Unterteil (72) vom Mittelteil (71) durch einen ringförmigen Ansatz (64) getrennt ist und der untere Hohlzylinder (5) am inneren Umfang Lamellen (51) mit einem kleineren Innendurchmesser als der Aussendurchmesser des ringförmigen Ansatzes (64) aufweist, so dass der untere Hohlzylinder (5) vom oberen Ende (32) her über den Körper (3) einschiebbar ist und die Lamellen (51) über den ringförmigen Ansatz (64) einschnappbar sind.

**20.** Vorrichtung nach einem der Ansprüche 1 bis 19, **dadurch gekennzeichnet, dass** die Kompressibilität der Kammer (34) durch gegen das untere Ende (31) offene Schlitze (37) im Unterteil (72) gegeben ist.

**21.** Vorrichtung nach einem der Ansprüche 1 bis 20, **dadurch gekennzeichnet, dass** die Kammer (34) einen Durchmesser d aufweist und als Kugelschicht mit einer Höhe x < d ausgebildet ist.

22. Vorrichtung nach Anspruch 21, **dadurch gekennzeichnet, dass** die senkrecht zur Zentralachse (33) stehende Grosskreisebene der Kugelschicht vom oberen Ende (38) der Kammer (34) einen Abstand y aufweist und dieser Abstand y < x ist.

23. Vorrichtung nach einem der Ansprüche 6 bis 22 mit einem Längsträger (1), **dadurch gekennzeichnet, dass** die Höhe H des grösseren Mantelsektors (63) der Summe H = D + A entspricht, wobei D der Durchmesser des Längsträgers ist; und A dem Abstand zwischen dem Dach (64) der Durchgangsöffnung (61) und der oberen Grundfläche (65) des oberen Hohlzylinders (6) entspricht.

24. Vorrichtung nach Anspruch 23, **dadurch gekennzeichnet, dass** die Höhe H des grösseren Mantelsektors (63) nur in einem Sektorbereich $\beta$ von 5° bis 20° der Summe H = D + A entspricht, wobei der Sektorbereich $\beta$ symmetrisch zum senkrecht zur Längsachse (87) der Durchgangsöffnung (61) liegenden Durchmesser der Hülse (6) angeordnet ist.

25. Vorrichtung nach einem der Ansprüche 1 bis 24, **dadurch gekennzeichnet, dass** sie zusätzlich ein Spannmittel (4) umfasst, welches den oberen Hohlzylinder (6) relativ zum Körper (3) nach unten bewegen kann.

26. Vorrichtung nach Anspruch 25, **dadurch gekennzeichnet, dass** die Höhe H des grösseren Mantelsektors (63) nur in einem Sektorbereich $\beta$ der Summe H = D + A entspricht, wobei der Sektorbereich $\beta$ so gewählt ist, dass das Verhältnis Fh / Fm des im Sektorbereich $\beta$ auf die obere Hülse (6) wirkenden Kraftanteils Fh zu der durch das Spannmittel (4) aufgebrachten Kompressionskraft Fm zwischen 0,15 und 0,35 beträgt.

27. Vorrichtung nach Anspruch 25, **dadurch gekennzeichnet, dass** die Höhe H des grösseren Mantelsektors (63) nur in einem Sektorbereich $\beta$ der Summe H = D + A entspricht, wobei der Sektorbereich $\beta$ so gewählt ist, dass das Verhältnis $F_h / F_m$ des im Sektorbereich $\beta$ auf die obere Hülse (6) wirkenden Kraftanteils $F_h$ zu der durch das Spannmittel (4) aufgebrachten Kompressionskraft $F_m$ zwischen 0,2 und 0,3 beträgt.

28. Vorrichtung nach einem der Ansprüche 1 bis 29, **dadurch gekennzeichnet, dass** sie zusätzlich ein Knochenfixationsmittel (2) umfasst.

29. Vorrichtung nach Anspruch 28, **dadurch gekennzeichnet, dass** der Kopf (21) des Knochenfixationsmittels (2) mit einer Strukturierung versehen ist.

30. Vorrichtung nach Anspruch 28 oder 29, **dadurch gekennzeichnet, dass** das Knochenfixationsmittel (2) eine Knochenschraube ist, deren Kopf (21) mit einem Innensechskant (23) versehen ist.

31. Vorrichtung nach einem der Ansprüche 1 bis 30, **dadurch gekennzeichnet, dass** der Körper (3) eine konzentrisch zur Zentralachse (33) verlaufende Durchgangsbohrung (74) aufweist.

32. Vorrichtung nach einem der Ansprüche 1 bis 31, **dadurch gekennzeichnet, dass** die Kammer (34) innen mit einer Strukturierung versehen ist.

33. Vorrichtung nach Anspruch 32, **dadurch gekennzeichnet, dass** der Kopf (21) des Knochenfixationsmittels (2) mindestens im Bereich der Strukturierung der Kammer (34) eine Zone aufweist, die weicher ist als die strukturierte Oberfläche der Kammer (34).

34. Vorrichtung nach einem der Ansprüche 1 bis 33, **dadurch gekennzeichnet, dass** der untere Hohlzylinder (5) unten innen und der Unterteil (72) aussen am unteren Ende (31) zylindrisch ausgebildet sind, wobei die Durchmesser, die Höhe und die Position dieser zylindrischen Teile (75;76) derart aufeinander abgestimmt sind, dass bei nicht angezogenem Spannmittel (4) ein Aufweiten des Unterteils (72) durch den zylindrischen Teil (76) im unteren Hohlzylinder (5) verhindert wird.

35. Vorrichtung nach einem der Ansprüche 24 oder 28 bis 34, **dadurch gekennzeichnet, dass** die Höhe H des grösseren Mantelsektors (63) nur in einem Sektorbereich $\beta$ der Summe H = D + A entspricht, wobei der Sektorbereich so gewählt ist, dass das Verhältnis zwischen dem Abstand (e), welcher durch die auf dem senkrecht zur Längsachse (87) der Durchgangsöffnung (61) stehenden Durchmesser der oberen Hülse (6) gemessene Länge zwischen dem Angriffspunkt der beim Anziehen des Spannmittels (4) mit der Kompressionskraft $F_m$ resultierenden Kraft $F_s$ auf den Längsträger (1) und der Zentralachse (33) gegeben ist, und dem Abstand (f), welcher durch die auf dem senkrecht zur Längsachse (87) der Durchgangsöffnung (61) stehenden Durchmesser der oberen Hülse (6) gemessene Länge zwischen der Zentralachse (33) und dem Angriffspunkt der beim Anziehen des Spannmittels (4) mit der Kompressionskraft $F_m$ resultierenden Kräfte $F_h$ auf den Sektorbereich $\beta$ gegeben ist, zwischen 0,15 und 0,5 beträgt.

36. Vorrichtung nach Anspruch 35, **dadurch gekennzeichnet, dass** die Höhe H des grösseren Mantelsektors (63) nur in einem Sektorbereich β der Summe H = D + A entspricht, wobei der Sektorbereich β so gewählt ist, dass das Verhältnis. zwischen dem Abstand (e), welcher durch die auf dem senkrecht zur Längsachse (87) der Durchgangsöffnung (61) stehenden Durchmesser der oberen Hülse (6) gemessene Länge zwischen dem Angriffspunkt der beim Anziehen des Spannmittels (4) mit der Kompressionskraft $F_m$ resultierenden Kraft $F_s$ auf den Längsträger (1) und der Zentralachse (33) gegeben ist, und dem Abstand (f), welcher durch die auf dem senkrecht zur Längsachse (87) der Durchgangsöffnung (61) stehenden Durchmesser der oberen Hülse (6) gemessene Länge zwischen der Zentralachse (33) und dem Angriffspunkt der beim Anziehen des Spannmittels (4) mit der Kompressionskraft $F_m$ resultierenden Kräfte $F_h$ auf den Sektorbereich β gegeben ist, zwischen 0,25 und 0,35 beträgt.

**Claims**

1. A device for connecting a longitudinal support (1) with a bone fixation device (2) provided with a spherical head (21), having

    A) a cylindrical body (3) with a central axis (33), comprising an upper section (70) with an upper end (32), a central section (71) and a lower section (72) wit a lower end (31), wherein

        a) the lower section (72) is provided with a spherical and compressible chamber (34) open on the underside for receiving a spherical head (21)

        b) the upper section (70) is provided with a means (35) for receiving a tensioning device (4) for the longitudinal support (1), and

        c) the central section (71) is provided with a hole (36) for receiving the longitudinal support (1) which hole runs perpendicular to its central axis (33)

    B) a lower hollow cylinder (5) arranged concentrically to the body (3) which can be shoved from the top end (32) over the bottom section (72), wherein the minimum internal diameter of the lower hollow cylinder (5) is less than the maximum external diameter of the bottom section (72), so that the bottom section (72) with the chamber (34) can be compressed by the lower hollow cylinder (5) which can be pushed downward and a spherical head (21) located in the chamber (34) can be fixed in rotative direction,

    C) an upper lower hollow cylinder (6) arranged concentrically to the body (3) with a passage opening (61) corresponding to the hole (36), open on the underside, running perpendicular to the central axis (33), and intended for partial surrounding of the longitudinal support (1)

    D) the lower hollow cylinder (5) can be pushed downward by means of a longitudinal support (1) positioned in the hole (36) and the passage opening (61) through the effect of the tensioning device (4) on the upper hollow cylinder (6), **characterised in that**

    E) a means (85) is provided at the lower end (84) of the upper hollow cylinder (6) and/or at the upper end (83) of the bottom hollow cylinder (5), which between the neighbouring front faces (81, 82) of the upper (6) and the lower hollow cylinder (5), besides two contact points of a longitudinal support (1) positioned in the hole (36) and the passage opening (61), form at least a third contact point, so that the effect of the tensioning device (4) on the upper hollow cylinder (6) means that the lower hollow cylinder (5) can be pushed downward coaxial to the central axis (33), and

    F) the external wall (86) of the bottom section (72) is formed with spherical zones.

2. Device in accordance with claim 1, **characterised in that** the external wall (86) of the bottom section (72) has a convex form.

3. Device in accordance with claims 1 or 2, **characterised in that** the means (85) is arranged is such a way that the two contact points of a longitudinal support (1) positioned in the hole (36) and in the passage opening (61) and at least the third contact point of the means (85) create a resulting force application point lying at a minimum distance to the central axis (33).

4. Device in accordance with one of the claims 1 to 3, **characterised in that** the hole (36) for receiving the longitudinal support (1) is attached eccentric to the central axis (33) in the body (3).

5. Device in accordance with one of the claims 1 to 4, **characterised in that** the hole (36) for is open at the side of the body (3).

6. Device in accordance with one of the claims 1 to 5, **characterised in that** the device (85) consists of the passage opening (61) dividing the upper hollow cylinder (6) into two shell sectors (62, 63) with different heights, wherein the shell sector (62) intended for closing the laterally open hole (36) of the body (3) has a lower height than the diametrically opposite shell sector (63).

7. Device in accordance with claim 1, **characterised in that** the shell sector (63) of the upper hollow cylinder (6) shows at least in a sector range β of 10° to 20° arranged symmetrically to the passage opening (61) has a height greater than the shell sector (62) intended for closing the laterally open hole (36) of the body (3).

8. Device in accordance with claim 6 or claim 7, **characterised in that** the difference in height of the two shell sectors (62, 63) is preferably in the range of 0.3 - 1.0 mm.

9. Device in accordance with one of the claims 4 to 8, **characterised in that** the passage opening (61) is arranged eccentric to the central axis (33) so that the shell sector (62) intended for closing the laterally open hole (36) of the body (3) is in section smaller than the diametrically opposite shell sector.

10. Device in accordance with one of the claims 1 to 9, **characterised in that** the inner cone surface of the lower hollow cylinder (5) has a form different from the external wall (86) of the lower section (72), so that when the hollow cylinder (5) is shoved on concentrically, only a circular contact to the spherical external wall (86) of the bottom section (72) is formed.

11. Device in accordance with one of the claims 1 to 9, **characterised in that** the inner cone surface of the lower hollow cylinder (5) has a form different from the external wall (86) of the lower section (72), so that when the hollow cylinder (5) is shoved on concentrically, only a circular zone contact to the spherical external wall (86) of the bottom section (72) is formed.

12. Device in accordance with claim 11, **characterised in that** the zone height of the circular zone contact surface between the inner shell surface of the lower hollow cylinder (5) and the external wall (86) of the bottom section (72) measures a maximum of 2 mm.

13. Device in accordance with one of the claims 1 to 12, **characterised in that** the height of the lower hollow cylinder (5) is dimensioned so that the longitudinal support (1) laid in the hole (36) and in the passage opening (61) cannot reach the floor of the hole (36) when the tensioning device (4) is activated.

14. Device in accordance with one of the claims 1 to 13, **characterised in that** the centre (73) of this spherical segment type external wall of the bottom section (72) lies below the centre (39) of the spherical chamber (34), wherein the term below means closer to the lower end (31) of the body (3).

15. Device in accordance with one of the claims 1 to 14, **characterised in that** the device (35) is an external thread that can receive a nut as tensioning device (4).

16. Device in accordance with one of the claims 1 to 14, **characterised in that** the device (35) is an internal thread that can receive a screw as tensioning device (4).

17. Device in accordance with one of the claims 1 to 16, **characterised in that** the bone fixation device (2) is a bone screw or a hook.

18. Device in accordance with one of the claims 1 to 17, **characterised in that** the chamber (34) is designed in such a way that the screw shaft (22) of the bone screw (2) connected to the spherical head (21) is locked with an angle of α from - 25° to + 25° in relation to the central axis (33).

19. Device in accordance with one of the claims 1 to 18, **characterised in that** the bottom section (72) is separated from the middle section (71) by a ring-shaped shoulder (64), and the lower hollow cylinder (5) is provided with lamellas (51) in the inner circumference with an internal diameter less than the external diameter of the ring-shaped shoulder (64), so that the lower hollow cylinder (5) can be shoved from the top end (32) over the body (3), and the lamellas (51) can latch over the ring-shaped shoulder (64).

20. Device in accordance with one of the claims 1 to 19, **characterised in that** the compression feature of the chamber (34) is provided by slots (37) in the bottom section (72) that are open towards the lower end (31).

21. Device in accordance with one of the claims 1 to 20, **characterised in that** the chamber (34) has a diameter d and is formed as a spherical segment with a height x < d.

22. Device in accordance with claim 21, **characterised in that** the great-circle surface of the spherical segment standing vertically to the central axis (33) and has a distance y from the upper end (38) of the chamber (34), and this distance is y < x.

23. Device in accordance with one of the claims 6 to 22 with a longitudinal support (1), **characterised in that** the height H of the larger shell sector (63) corresponds to the total H = D + A, with D being the diameter of the longitudinal support (1) and A being the distance between the roof (64) of the passage opening (61) and the upper base surface (65) of the upper hollow cylinder (6).

24. Device in accordance with claim 23, **characterised in that** the height H of the larger shell sector (63) corresponds to the total H = D + A only in a sector range β of 5° to 20°, wherein the sector range β is arranged symmetrically to the diameter of the bush (6) lying vertically to the longitudinal support (87) of the passage opening (61).

25. Device in accordance with one of the claims 1 to 24, **characterised in that** it is additionally provided with a tensioning device (4) which can move the upper hollow cylinder (6) downwards relative to the body (3)

26. Device in accordance with claim 25, **characterised in that** the height H of the larger shell sector (63) corresponds to the total H = D + A only in a sector range β, wherein the sector range β is chosen so that the ratio $F_h / F_m$ of the force section $F_h$ affecting the upper bush (6) in the sector range β to the compression force $F_m$ applied by the tensioning device (4) measures between 0.15 and 0.35.

27. Device in accordance with claim 25, **characterised in that** the height H of the larger shell sector (63) corresponds to the total H = D + A only in a sector range β, wherein the sector range β is chosen so that the ratio $F_h / F_m$ of the force section $F_h$ affecting the upper bush (6) in the sector range β to the compression force $F_m$ applied by the tensioning device (4) measures between 0.2 and 0.3.

28. Device in accordance with one of the claims 1 to 29, **characterised in that** it also comprises a bone fixation device (2).

29. Device in accordance with claim 28, **characterised in that** the head (21) of the bone fixation device (2) is provided with structuring.

30. Device in accordance with claim 28 or 29, **characterised in that** the bone fixation device (2) is a bone screw, whose head (21) is provided with a hexagon socket (23).

31. Device in accordance with one of the claims 1 to 30, **characterised in that** the body (3) is provided with a passage opening (74) running concentrically to the central axis (33).

32. Device in accordance with one of the claims 1 to 31, **characterised in that** the chamber (34) is provided with structuring on the inside.

33. Device in accordance with claim 32, **characterised in that** head (21) of the bone fixation device (2) is provided with a zone at least in the structuring of the chamber (34) that is softer than the structured surface of the chamber (34).

34. Device in accordance with one of the claims 1 to 33, **characterised in that** the lower hollow cylinder (5) is formed as a cylinder at the bottom interior side, as is the bottom section (72) on the outside at the lower end (31), wherein the diameter, the height and the position of these cylindrical parts (75, 76) are co-ordinated in such a way that when the tensioning nut (4) is not tightened, the bottom section (72) is prevented from being expanded by the cylindrical section (76) in the lower hollow cylinder (5).

35. Device in accordance with one of the claims 24 or 28 to 34, **characterised in that** the height H of the larger shell sector (63) corresponds to the total H = D + A only in a sector range β of 5° to 20°, wherein the sector range β is chosen so that the ratio between the distance (e) caused by the length - measured on the diameter of the upper bush (6) standing vertical to the longitudinal axis (87) of the passage opening (61) - between the contact point of the force $F_a$ on the longitudinal support (1) - resulting when the tensioning device (4) is tightened with the compression force $F_m$ - and the central axis (33), and the distance (f) which because of the length - measured on the diameter of the upper bush (6) standing vertical to the longitudinal axis (87) of the passage opening (61) - between the central axis (33) and the contact point of the force $F_h$ on the sector range β resulting when the tensioning device (4) is tightened with the compression force $F_m$, measures between 0.15 and 0.5.

36. Device in accordance with claim 35, **characterised in that** the height H of the larger shell sector (63) corresponds to the total H = D + A only in a sector range β of 5° to 20°, wherein the sector range β is chosen so that the ratio between the distance (e) caused by the length - measured on the diameter of the upper bush (6) standing vertical to the longitudinal axis (87) of the passage opening (61) - between the contact point of the force $F_a$ on the longitudinal support (1) - resulting when the tensioning device (4) is tightened with the compression force $F_m$ - and the central axis (33), and the distance (f) which because of the length - measured on the diameter of the upper bush (6) standing vertical to the longitudinal axis (87) of the passage opening (61) - between the central axis (33) and the contact point of the force $F_h$ on the sector range β resulting when the tensioning device (4) is tightened with the compression force $F_m$, measures between 0.25 and 0.35.

**Revendications**

1. Dispositif pour relier un support longitudinal (1) à un organe de fixation des os (2) présentant une tête sphérique (21) dans un dispositif de fixation de la colonne vertébrale, avec

   A) un corps cylindrique (3) avec un axe central (33) comprenant une partie supérieure (70) avec une extrémité supérieure (32), une partie centrale (71) et une partie inférieure (72) avec une extrémité inférieure (31),

   a) la partie inférieure (72) étant équipée d'une chambre (34) sphérique comprimable et ouverte vers le bas, destinée à recevoir la tête sphérique (21);
   b) la partie supérieure (70) présentant des organes (35) de réception d'un organe de serrage (4) pour le support longitudinal (1); et
   c) la partie centrale (71) présentant un alésage (36) transversal par rapport à l'axe central (33) et destiné à recevoir le support longitudinal (1);

   B) un cylindre creux inférieur (5) disposé de façon concentrique par rapport au corps (3) et pouvant glisser sur la partie inférieure (72) depuis l'extrémité supérieure (32), le diamètre interne minimal du cylindre creux inférieur (5) étant plus petit que le diamètre externe maximal de la partie inférieure (72), de sorte que la partie inférieure (72) avec la chambre (34) est comprimable par le cylindre creux inférieur (5) pouvant glisser vers le bas et qu'une tête sphérique (21) se trouvant dans la chambre (34) peut être bloquée dans le sens rotatif;
   C) un cylindre creux supérieur (6) disposé de façon concentrique par rapport au corps (3) avec un trou traversant (61) correspondant à l'alésage (36), ouvert vers le bas, transversal par rapport à l'axe central (33) et destiné à enserrer partiellement le support longitudinal (1);
   D) le cylindre creux inférieur (5) pouvant glisser vers le bas au moyen d'un support longitudinal (1) introduit dans l'alésage (36) et dans le trou traversant (61), par l'action de l'organe de serrage (4) sur le cylindre creux supérieur (6), **caractérisé en ce que**
   E) sur l'extrémité inférieure (84) du cylindre creux supérieur (6) et/ou sur l'extrémité supérieure (83) du cylindre creux inférieur (5), des organes (85) sont prévus formant au moins un troisième point de contact entre les surfaces frontales voisines (81;82) des cylindres creux supérieur (6) et inférieur (5), en plus des deux points de contact d'un support longitudinal (1) introduit dans l'alésage (36) et dans le trou traversant (61), de sorte que par l'action de l'organe de serrage (4) sur le cylindre creux supérieur (6), le cylindre creux inférieur (5) peut glisser vers le bas de façon coaxiale par rapport à l'axe central (33); et que
   F) la paroi extérieure (86) de la partie inférieure (72) a la forme d'un segment sphérique à deux bases.

2. Dispositif selon la revendication 1, **caractérisé en ce que** la paroi extérieure (86) de la partie inférieure (72) a une forme convexe.

3. Dispositif selon la revendication 1 ou 2, **caractérisé en ce que** les organes (85) sont disposés de façon à ce que les deux points de contacts d'un support longitudinal (1) introduit dans l'alésage (36) et le trou traversant (61) et le troisième point de contact au moins des organes (85) forment un point d'application des forces résultant situé à une distance minimale de l'axe longitudinal (33).

4. Dispositif selon une des revendications 1 à 3, **caractérisé en ce que** l'alésage (36) destiné à recevoir le support longitudinal (1) est disposé dans le corps (3) de façon excentrée par rapport à l'axe central (33).

5. Dispositif selon une des revendications 1 à 4, **caractérisé en ce que** l'alésage (36) sur le corps (3) est ouvert sur le côté.

6. Dispositif selon une des revendications 1 à 5, **caractérisé en ce que** les organes (85) consistent **en ce que** le trou traversant (61) sépare le cylindre creux supérieur (6) en deux secteurs de gaine (62; 63) de hauteur différente, le secteur de gaine (62) destiné à obturer l'alésage (36) ouvert sur le côté du corps (3) présentant une hauteur inférieure à celle du secteur de gaine (63) diamétralement opposé.

7. Dispositif selon la revendication 6, **caractérisé en ce que** le secteur de gaine (63) du cylindre creux supérieur (6) présente, au moins dans une zone sectorielle β de 10° à 20° placée symétriquement par rapport au trou traversant (61), une hauteur supérieure à celle du secteur de gaine (62) destiné à obturer l'alésage (36) ouvert sur le côté du corps (3).

8. Dispositif selon la revendication 6 ou 7, **caractérisé en ce que** la hauteur différente des deux secteurs de gaine (62,63) se situe dans une plage de 0,3 à 1,0 mm.

9. Dispositif selon une des revendications 4 à 8, **caractérisé en ce que** le trou traversant (61) est excentré par rapport à l'axe central (33), de sorte que le secteur de gaine (62) destiné à obturer l'alésage (36) ouvert sur le côté du corps (3) est sectoriellement plus petit que le secteur de gaine (63) diamétralement opposé.

10. Dispositif selon une des revendications 1 à 9, **caractérisé en ce que** la surface de gaine interne du cylindre creux inférieur (5) présente une forme différente de la paroi extérieure (86) de la partie inférieure (72), de sorte que lors du chevauchement concentrique du cylindre creux (5), il s'établit uniquement un contact de forme circulaire avec la paroi extérieure (86) de la partie inférieure (72).

11. Dispositif selon une des revendications 1 à 9, **caractérisé en ce que** la surface de gaine interne du cylindre creux inférieur (5) présente une forme différente de la paroi extérieure (86) de la partie inférieure (72), de sorte que lors du chevauchement concentrique du cylindre creux (5), il s'établit uniquement un contact en forme de segment circulaire avec la paroi extérieure (86) de la partie inférieure (72).

12. Dispositif selon la revendication 11, **caractérisé en ce que** la hauteur de segment de la surface de contact en forme de segment circulaire entre la surface de gaine interne du cylindre creux inférieur (5) et la paroi extérieure (86) de la partie inférieure (72) est de 2 mm au maximum.

13. Dispositif selon une des revendications 1 à 12, **caractérisé en ce que** la hauteur du cylindre creux inférieur (5) est conçue de façon à ce que le support longitudinal (1) introduit dans l'alésage (36) et dans le trou traversant (61) ne puisse pas atteindre le fond de l'alésage (36) lorsque l'on actionne l'organe de serrage (4).

14. Dispositif selon une des revendications 1 à 13, **caractérisé en ce que** le centre (73) de la paroi extérieure de la partie inférieure (72), en forme de segment sphérique à deux bases, se situe en dessous du centre (39) de la chambre (34) sphérique, "en dessous" signifiant ici "plus près de l'extrémité inférieure (31) du corps (3)".

15. Dispositif selon une des revendications 1 à 14, **caractérisé en ce que** l'organe (35) est un filetage mâle pouvant recevoir un écrou comme organe de serrage (4).

16. Dispositif selon une des revendications 1 à 14, **caractérisé en ce que** l'organe (35) est un filetage femelle pouvant recevoir une vis comme organe de serrage (4).

17. Dispositif selon une des revendications 1 à 16, **ca-**

**ractérisé en ce que** l'organe de fixation des os (2) est une vis pour ostéosynthèse ou un crochet.

18. Dispositif selon une des revendications 1 à 17, **caractérisé en ce que** la chambre (34) est conçue de telle sorte que la tige (22) de la vis pour ostéosynthèse (2) qui touche la tête sphérique (21) peut être bloquée dans un angle $\alpha$ de -25° à +25° par rapport à l'axe central (33).

19. Dispositif selon une des revendications 1 à 18, **caractérisé en ce que** la partie inférieure (72) est séparée de la partie centrale (71) par un insert circulaire (64) et que le cylindre creux inférieur (5) est muni sur sa circonférence intérieure de lamelles (51) dont le diamètre interne est plus petit que le diamètre externe de l'insert circulaire (64), de sorte que l'on peut introduire le cylindre creux inférieur (5) sur le corps (3) par l'extrémité supérieure (32) et que les lamelles (51) peuvent se bloquer par encliquetage sur l'insert circulaire (64).

20. Dispositif selon une des revendications 1 à 19, **caractérisé en ce que** la compressibilité de la chambre (34) est obtenue par des fentes (37) dans la partie inférieure (72), ouvertes en direction de l'extrémité inférieure (31).

21. Dispositif selon une des revendications 1 à 20, **caractérisé en ce que** la chambre (34) présente un diamètre d et a la forme d'un segment sphérique à deux bases de hauteur x < d.

22. Dispositif selon la revendication 21, **caractérisé en ce que** le plan du grand cercle du segment sphérique à deux bases est perpendiculaire à l'axe central (33) et se trouve à une distance y de l'extrémité supérieure (38) de la chambre (34), cette distance étant y < x.

23. Dispositif selon une des revendications 6 à 22 avec un support longitudinal (1), **caractérisé en ce que** la hauteur H du secteur de gaine (63) le plus grand correspond à la somme H = D + A, D étant le diamètre du support longitudinal; et A correspondant à l'écart entre le toit (64) du trou traversant (61) et la surface de base supérieure (65) du cylindre creux supérieur (6).

24. Dispositif selon la revendication 23, **caractérisé en ce que** la hauteur H du secteur de gaine (63) le plus grand ne correspond à la somme H = D + A que dans une zone sectorielle $\beta$ de 5° à 20°, la zone sectorielle $\beta$ étant placée symétriquement par rapport au diamètre du manchon (6), diamètre lui-même perpendiculaire à l'axe longitudinal (87) du trou traversant (61).

**25.** Dispositif selon une des revendications 1 à 24, **caractérisé en ce qu'**il comprend un organe de serrage (4) supplémentaire pouvant déplacer le cylindre creux supérieur (6) vers le bas par rapport au corps (3).

**26.** Dispositif selon la revendication 25, **caractérisé en ce que** la hauteur H du secteur de gaine (63) le plus grand ne correspond à la somme H = D + A que dans une zone sectorielle $\beta$, la zone sectorielle $\beta$ étant choisie de façon à ce que le rapport $F_h/F_m$ entre la part de la force $F_h$ agissant sur le manchon supérieur (6) dans la zone sectorielle $\beta$ et la force de compression $F_m$ exercée par l'organe de serrage (4) se situe entre 0,15 et 0,35.

**27.** Dispositif selon la revendication 25, **caractérisé en ce que** la hauteur H du secteur de gaine (63) le plus grand ne correspond à la somme H = D + A que dans une zone sectorielle $\beta$, la zone sectorielle $\beta$ étant choisie de façon à ce que le rapport $F_h/F_m$ entre la part de la force $F_h$ agissant sur le manchon supérieur (6) dans la zone sectorielle $\beta$ et la force de compression $F_m$ exercée par l'organe de serrage (4) se situe entre 0,2 et 0,3.

**28.** Dispositif selon une des revendications 1 à 29, **caractérisé en ce qu'**elle comprend en plus un organe de fixation des os (2).

**29.** Dispositif selon la revendication 28, **caractérisé en ce que** la tête (21) de l'organe de fixation des os (2) est munie d'une structuration.

**30.** Dispositif selon la revendication 28 ou 29, **caractérisé en ce que** l'organe de fixation des os (2) est une vis pour ostéosynthèse dont la tête (21) est munie d'un six pans creux (23).

**31.** Dispositif selon une des revendications 1 à 30, **caractérisé en ce que** le corps (3) présente un trou traversant (74) concentrique par rapport à l'axe longitudinal (33).

**32.** Dispositif selon une des revendications 1 à 31, **caractérisé en ce que** la chambre (34) est munie d'une structuration à l'intérieur.

**33.** Dispositif selon la revendication 32, **caractérisé en ce que** la tête (21) de l'organe de fixation des os (2) présente, au moins dans le secteur de la structuration de la chambre (34), une zone plus douce que la surface structurée de la chambre (34).

**34.** Dispositif selon une des revendications 1 à 33, **caractérisé en ce que** l'intérieur du bas du cylindre creux inférieur (5) et l'extérieur de la partie inférieure (72) sur l'extrémité inférieure (31) sont de forme cylindrique, les diamètres, la hauteur et la position de ces pièces cylindriques (75;76) étant ajustés les uns par rapport aux autres de telle sorte que lorsque l'organe de serrage (4) n'est pas serré, cela évite un élargissement de la partie inférieure (72) par la partie cylindrique (76) dans le cylindre creux inférieur (5).

**35.** Dispositif selon une des revendications 24 ou 28 à 34, **caractérisé en ce que** la hauteur H du secteur de gaine (63) le plus grand ne correspond à la somme H = D + A que dans une zone sectorielle $\beta$, la zone sectorielle étant choisie de façon à ce que le rapport entre l'écart (e) et l'écart (f) se situe entre 0,15 et 0,5; l'écart (e) étant donné par la longueur mesurée sur le diamètre du manchon supérieur (6), perpendiculaire à l'axe longitudinal (87) du trou traversant (61), entre l'axe central (33) et le point d'application de la force $F_s$ sur le support longitudinal (1), résultant du serrage de l'organe de serrage (4) par la force de compression $F_m$, et l'écart (f) étant donné par la longueur mesurée sur le diamètre du manchon supérieur (6), perpendiculaire à l'axe longitudinal (87) du trou traversant (61), entre l'axe central (33) et le point d'application des forces $F_h$ sur la zone sectorielle $\beta$, résultant du serrage de l'organe de serrage (4) par la force de compression $F_m$.

**36.** Dispositif selon la revendication 35, **caractérisé en ce que** la hauteur H du secteur de gaine (63) le plus grand ne correspond à la somme H = D + A que dans une zone sectorielle $\beta$, la zone sectorielle $\beta$ étant choisie de façon à ce que le rapport entre l'écart (e) et l'écart (f) se situe entre 0,25 et 0,35; l'écart (e) étant donné par la longueur mesurée sur le diamètre du manchon supérieur (6), perpendiculaire à l'axe longitudinal (87) du trou traversant (61), entre l'axe central (33) et le point d'application de la force $F_s$ sur le support longitudinal (1), résultant du serrage de l'organe de serrage (4) par la force de compression $F_m$, et l'écart (f) étant donné par la longueur mesurée sur le diamètre du manchon supérieur (6), perpendiculaire à l'axe longitudinal (87) du trou traversant (61), entre l'axe central (33) et le point d'application des forces $F_h$ sur la zone sectorielle $\beta$, résultant du serrage de l'organe de serrage (4) par la force de compression $F_m$.

Fig. 1

Fig. 2

Detail B

Fig. 3

Fig. 4

Fig. 5

Fig. 6